**Europäisches Patentamt**

**European Patent Office**

**Office européen des brevets**

(19)

(11) Publication number: **0 204 311**
**B1**

(12)

# EUROPEAN PATENT SPECIFICATION

(45) Date of publication of the patent specification:
06.09.89

(51) Int. Cl.⁴: **F 16 L 19/02, F 16 B 39/284**

(21) Application number: **86107510.9**

(22) Date of filing: **03.06.86**

(54) Non-loosening leur nut.

(30) Priority: **03.06.85 US 740353**

(43) Date of publication of application:
**10.12.86 Bulletin 86/50**

(45) Publication of the grant of the patent:
**06.09.89 Bulletin 89/36**

(84) Designated Contracting States:
**DE FR GB**

(56) References cited:
**BE-A- 535 526**
**CH-A- 248 339**
**DE-A- 2 557 130**
**GB-A- 1 397 493**
**GB-A- 2 024 974**
**GB-A- 2 089 921**
**GB-A- 2 146 405**

(73) Proprietor: **Hewlett-Packard Company, Mail Stop 20 B-O 3000 Hanover Street, Palo Alto California 94304 (US)**

(72) Inventor: **Stephens, Thomas P., 420 Ipswich Road, Boxford Massachusetts 01921 (US)**

(74) Representative: **Liesegang, Roland, Dr.-Ing., FORRESTER & BOEHMERT Widenmayerstrasse 4 Postfach 22 01 37, D-8000 München 22 (DE)**

## Description

This invention relates to a Luer nut assembly comprising the features of the pre-characterizing part of claim 1.

In a known Luer nut assembly of this kind (GB-A-2 089 921) the longitudinal extension has a circular cross section and is rendered resilient by a pair of opposite axial slots.

A known torque locknut has a polygonal cross section which is locally weakened by radially inwardly directed deformations rendering the inner threaded surface of the nut polygonal and at the same time providing the required elasticity (US-A-3 456 704).

The normal Luer nut has a 10-pitch thread with 2 starts. Less than one turn of the nut is required to couple or decouple the tubes. Unfortunately, however, the nut is often loosened during normal handling or just relaxing and the seal broken. This is due to the high angular contact of the threads. The thread contact point is a steep wedge that is likely to unloosen because of the steep angle of contact. Some improvement was attempted by reducing the diameter of the threads as the interior of the nut was approached so as to increase the frictional torque as the nut was screwed into position. In some assemblies, however, the dimension of the threads in the nut and of the annular projection at the end of the female tube, although within normal manufacturing tolerances, were such that the frictional torque became too great for an operator to easily overcome before the male tube seated firmly enough in the female tube to form a seal. This is prevalent in a round female flange and is caused by the round shape of the nut being extended radially outward until the nut resists further radial expansion because the walls of the nut are in tension or hoop stress.

The problem underlying the invention is to provide a Luer nut assembly of the kind mentioned which on the one hand may be safely secured against loosening and on the other hand is not weakened by local slots or outer deformations.

This problem is accomplished by the features of claim 1.

In accordance with the present invention, the cross-section of the threaded section of the Luer nut is generally in the shape of a polygon that is deformable rather than stiff as in the prior art. In an axial view, the shape of the projection at the end of the female tube may be annular as in previous assemblies or it may have other shapes such as a rectangle. In any case, a portion of the periphery of the projection contacts one or more sides of the polygon. Wherever contact is made, the radial dimensions of the projection are such that the side is bent radially outward so that it acts like a spring and exerts a radially inward force on the projection. The resulting force of friction creates a frictional torque which does noct prevent the operator from turning the nut, but which is sufficient to prevent the nut from becoming loose during normal use.

If the male tube is nearly seated in the female tube when the projection of the female tube reaches a point where the nut can be received onto it, male tube and female tube will be held very neraly in axial alignment so that the periphery of the projection may contact a side of the polygon at only one point. Otherwise it would be necessary for the periphery to make contact with a number of sides of the polygon so that the femal tube is held in axial alignment with the nut. The latter structure is preferable in our case, however.

Other features and advantages can be recognized from the drawings and the description thereto.

Figure 1 shows what would be seen in an axial plane of a Luer nut assembly constructed in accordance with this invention at a point just before the male tube is seated in the female tube;

Figure 2 is an axial view of an assembly incorporating the invention in which the cross-section of the threaded portion of the Luer nut is generally triangular and the periphery of the projection at the end of the female tube is annular;

Figure 3 is an axial view of an assembly incorporating the invention in which the cross-section of the threaded portion of the Luer nut is generally triangular and the periphery of the projection at end of the female tube has two opposed arcs; and

Figure 4 is an axial view of an assembly incorporating the invention in which the cross-section of the threaded portion of the Luer nut is generally triangular and the periphery of the projection at the end of the female tube is approaching a square.

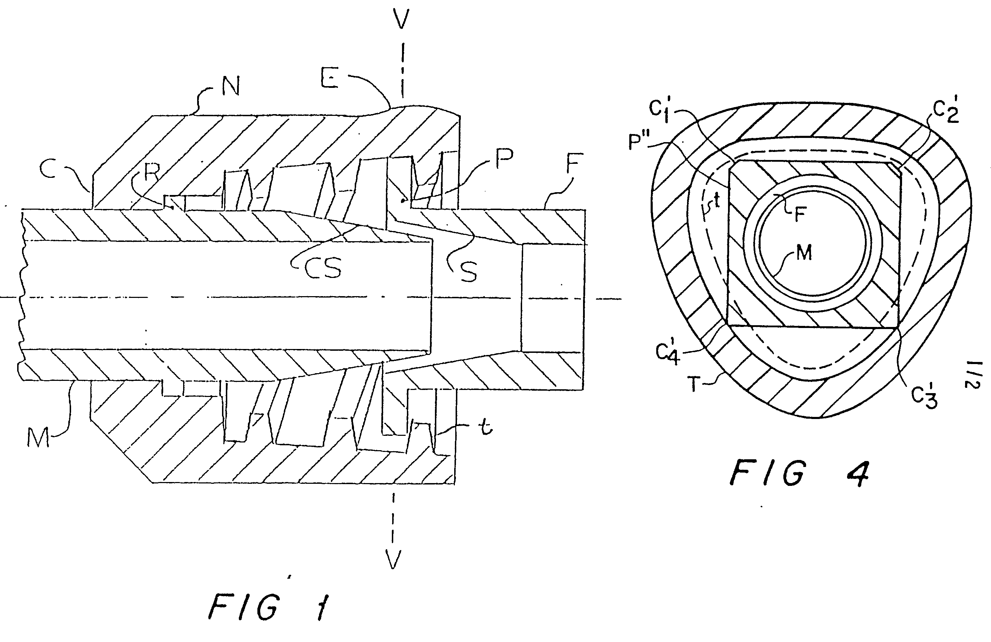

Figure 1 illustrates what would be in an axial plane of an assembly incorporating the invention. A hollow male tube M as an exterior conical surface CS and an annular ridge R adjacent the larger end of the surface CS. A hollow female tube F has an axial conical recess S into which the surface CS can be made to fit so as to form a seal and an outwardly extending projection P. A hollow Luer nut N is shown having a collar C at one end thereof and an internal thread t in the other end E that engages the projection P. As the nut N is rotated in one direction about its axis with respect to the female tube F, the projection P advances in the threads t and the female tube F is moved toward the male tube M. Finally, the conical surface CS and the conical recess S are seated as to form a seal.

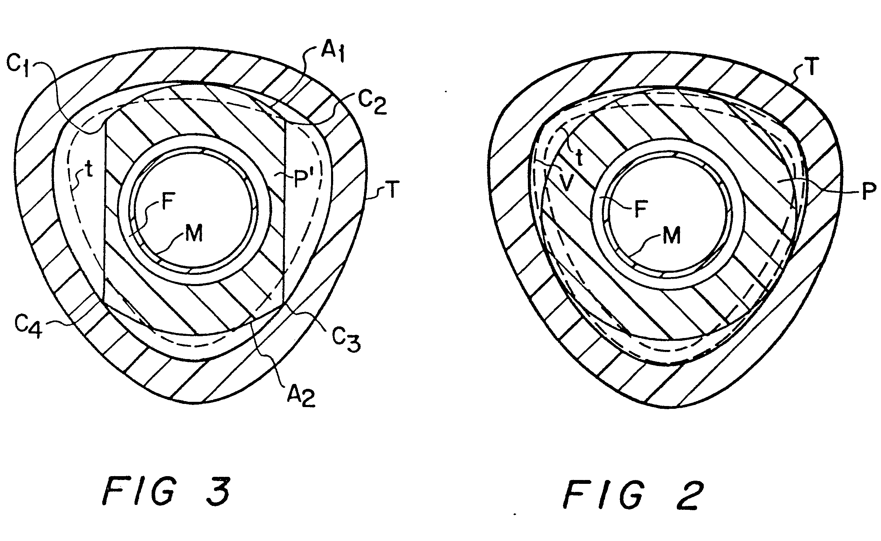

Reference is now made to Figure 2 which illustrates what is seen in an assembly in a plane that is perpendicular to the axis of the female tube F and which passes through the projection P. Inasmuch as Fiugre 1 is in itself a section, Figure 2 is not a section of Figure 1 but would be located at VV therein. In Figure 2, the cross-section of the end E of the nut N is a polygon generally in the form of an equilateral triangle T. The projection P is an annulus which contacts the midpoints of the iner sides of the triangle so as to bend the sides outward. The undistorted shape of the inner surface of the triangle T is shown by a dash-dot line V. Although not in the plane shown in Figure 2, the location of the threads t, when the sides of the triangle T are bent outward by the projection P, is shown by a dashed line. Where the periphery of the annular projection P contacts the sides of the triangle, it engages the threads t so that the nut N can be screwed onto the threads. The fact that the projection P is not engaged with the threads t at every point does not prevent this action from occurring.

An important aspect of this invention is the fact that wherever the periphery of the projection P on the female tube F contacts a side of the polygon formed inside the threaded end E of the nut N, its radial dimension is such as to bend the side outward. The side acts as a spring and creates a frictional torque sufficient to prevent the nut from being loosened during use.

In Figure 2, the polygon formed by the cross-section of the end E of the nut N is an equilateral triangle T with rounded corners. Sharp corners could be used if desired. The periphery of the projection P is annular and is of such radius as to contact the central points of the sides of the triangle T and bend the sides outward from their unstressed position shown by the dashed line V. The bending can also be seen in Figure 1 where the projection P contacts the inside of the threaded end E of the nut N.

In Figure 3, the polygon formed by the cross-section of the end E of the nut N is an equilateral triangle T as in Figure 2, but the periphery of the projection P is formed by two arcs $A_1$ and $A_2$ on opposite sides of the axis of the female tube F and having that axis as a center, and by two parallel lines on either side of the axis and intersecting the arcs at corners $C_1$, $C_2$, $C_3$ and $C_4$. The central portion of the arc $A_1$ contacts the center of the top side of the triangle T, and the corners $C_3$ and $C_4$ respectively contact the other sides at points below the center. The sides are bent out at the points of contact so as to create the frictional torque required. The dash-dot line V is not shown.

Figure 4 is the same as Figure 3 except that the periphery of the projection P″ is in the form of a square that contacts the triangular polygon T at its lower corners $C'_3$ and $C'_4$. The fact that the upper corners $C'_1$ and $C'_2$ do not initially contact the polygon presents no problem because the male and female tubes will stay substantially in alignment when they are nearly seated. Note, however, that the top side of the triangle T is not bent outward.

It is apparent that the outer surface of the end E of the nut N can have a cross-section that is shaped differently from that of its inner surface.

In order to increase the frictional torque as the nut is being screwed onto the external projection of the female tube, the radial dimensions of the polygon formed by the inner surface on which the threads are formed can be decreased as the interior of the nut is approached by an amount that is greater than that required to extricate the mold, but this should not be such as to cause binding.

## Claims

1. A Luer nut assembly comprising
   - a female hollow tube,
   - a male hollow tube and
   - a hollow nut, wherein
   a) said female tube (F) has
   - an exterior projection (P) at the end, said projection (P) having a given periphery in a plane perpendicular to the axis of the tube,
   - means defining a conical recess (S) in one end of said female tube (F),
   b) said male tube (M) has
   - an external surface (CS) at one end thereof that is conical in shape so as to fit into said conical recess (S) when the ends of said female and male tubes are axially drawn together,
   c) a shoulder (R) is formed on the exterior of said male tube (M) at a point adjacent the larger end of said exterior conical surface (CS),
   d) said hollow nut (N) has threads (t) formed at a longitudinal extension on an interior surface thereof about an axis of said nut (N),
   e) said hollow nut (N) has an inwardly extending collar (C) at the other end through which said male tube (M) passes, said collar (C) being more remote from the interior of said nut (N) than said shoulder (R), characterized in that
   f) the cross section of said nut (N) in a plane perpendicular to said axis in said longitudinal extension is polygonal,
   g) said longitudinal extension is elastically deformable in radial direction around its entire periphery, and
   h) the dimensions and shape of the periphery of said projection (P) are such that it contacts the interior surface of said longitudinal extension of said nut (N) at least at one point.

2. An assembly according to Claim 1 wherein the periphery of said exterior projection (P) is annular.

3. An assembly according to Claim 1 wherein the periphery of said exterior projection (P) is rectangular.

4. An assembly according to Claim 1 wherein the periphery of said exterior projection (P) is in the form of parallel lines connected at opposite ends by curved lines.

## Patentansprüche

1. Schrauben-Mutteranordnung der Luer-Bauart mit
   - einem weiblichen Rohr,
   - einem männlichen Rohr und einer
   - hohlen Mutter, wobei
   a) das weibliche Rohr (F) aufweist:
   - einen äusseren Vorsprung (P) an seinem Ende, wobei dieser Vorsprung in einer Ebene senkrecht zur Rohrachse einen vorgegebenen Umfang hat,
   - eine konische Aussparung (S) in einem Ende des weiblichen Rohres (F)
   b) das männliche Rohr (M) aufweist:
   - eine äussere Oberfläche (CS) an seinem einen Ende, die derart konisch ist, dass sie in die konische Aussparung (S) passt, wenn die Enden des weiblichen und des männlichen Rohres axial zueinander gezogen sind,

c) eine Schulter (R) an einer Stelle nahe dem grossen Ende der konischen Aussenfläche (CS) aussen auf dem männlichen Rohr (M) angeordnet ist,

d) die Mutter an der Innenfläche eines sich um eine Achse der Mutter (N) erstreckenden Längsteiles Gewindegänge (t) aufweist,

e) die Mutter (N) einen nach innen weisenden Kragen (C) an ihrem Ende aufweist, durch welches das männliche Rohr (M) hindurchpasst, wobei der Kragen (C) vom Inneren der Mutter (N) weiter entfernt ist, als die Schulter (R), dadurch gekennzeichnet, dass

f) der Querschnitt der Mutter (N) in einer Ebene senkrecht zur Achse ihres Längsteiles polygonal ist,

g) das Längsteil elastisch radial um seinen gesamten Umfang verformbar ist, und

h) Abmessungen und Gestalt des Umfangs des Vorsprungs (P) so beschaffen sind, dass er die Innenfläche des Längsteiles der Mutter (N) an mindestens einem Punkt berührt.

2. Anordnung nach Anspruch 1, bei der der Umfang des äusseren Vorsprungs (P) ringförmig ist.

3. Anordnung nach Anspruch 1, bei der der Umfang des äusseren Vorsprungs (P) rechteckig ist.

4. Anordnung nach Anspruch 1, bei der der Umfang des äusseren Vorsprungs (P) von Parallelen gebildet ist, die an entgegengesetzten Enden durch Bögen verbunden sind.

**Revendications**

1. Un assemblage à écrou Luer comprenant:
– un tube creux femelle,
– un tube creux mâle et
– un écrou creux,
dans lequel:
a) ledit tube femelle (F) comporte:
– une saillie extérieure (P) à l'extrémité, ladite saillie (P) comportant une périphérie donnée dans un perpendiculaire à l'axe du tube,

– un moyen définissant un évidement conique (S) dans une extrémité dudit tube femelle (F),
b) ledit tube mâle (M) comporte:
– à une extrémité une surface extérieure (CS) qui a une forme conique de façon à s'emboîter dans ledit évidement conique (S) quand les extrémités desdits tubes femelle et mâle sont axialement engagées l'une dans l'autre,

c) un épaulement (R) est formé sur l'extérieur dudit tube mâle (M) en un point adjacent à la grande extrémité de ladite surface conique extérieure (CS),

d) ledit écrou creux (N) comporte des filets (t) formés sur un prolongement longitudinal d'une surface intérieure dudit écrou autour d'un axe dudit écrou (N),

e) ledit écrou creux (N) comporte un collet (C) s'étendant vers l'intérieur à l'autre extrémité au travers de laquelle passe ledit tube mâle (M), ledit collet (C) étant plus éloigné de l'intérieur dudit écrou (N) que ledit épaulement (R), caractérisé en ce que:

f) la section droite dudit écrou (N) dans un plan perpendiculaire audit axe dans ledit prolongement longitudinal est polygonale,

g) ledit prolongement longitudinal est élastiquement déformable dans une direction radiale sur toute sa périphérie, et

h) les dimensions et la forme de la périphérie de ladite saillie (P) sont telles qu'elle entre en contact avec la surface intérieure dudit prolongement longitudinal dudit écrou (N) au moins en un point.

2. Un assemblage selon la revendication 1, dans lequel la périphérie de ladite saillie extérieure (P) est annulaire.

3. Un assemblage selon la revendication 1, dans lequel la périphérie de ladite saillie extérieure (P) est rectangulaire.

4. Un assemblage selon la revendication 1, dans lequel la périphérie de ladite saillie extérieure (P) se présente sous la forme de lignes parallèles reliées à des extrémités opposées par des lignes incurvées.

1/2

**FIG 4**

**FIG 1**

5

2/2

FIG 2

FIG 3